# EUROPEAN PATENT SPECIFICATION

(11) **EP 0 859 002 B1**
(45) Date of publication and mention of the grant of the patent: **02.07.2003**
(21) Application number: 98301090.1
(22) Date of filing: 13.02.1998
(51) Int. Cl.: C07D 277/20, C07D 277/44

(54) **Process for producing chloroacetylaminothiazoleacetic acid derivatives**
Verfahren zur Herstellung von Chloroacetylaminothiazoleaceticsäure Derivate
Procédé pour la préparation de derivés de l'acide acétique du chloracetylaminothiazole

(30) Priority: 13.02.1997 JP 2934897; 28.08.1997 JP 23287097
(43) Date of publication of application: 19.08.1998
(73) Proprietor: TOKUYAMA CORPORATION, Tokuyama-shi Yamaguchi-ken (JP)
(72) Inventor: Matsunaga, Tomonori, Tokuyama-shi, Yamaguchi-ken (JP); Iwasaki, Fumiaki, Tokuyama-shi, Yamaguchi-ken (JP); Hirota, Yoshihiro, Tokuyama-shi, Yamaguchi-ken (JP)
(74) Representative: Cresswell, Thomas Anthony

(56) References cited:
- GB-A- 1 580 622
- GB-A- 1 581 854
- GB-A- 1 599 722
- GB-A- 1 601 028
- US-A- 4 284 791
- US-A- 4 511 736
- HARDY ET AL: "The Chemistry of Some 2-Aminothiazol-4-ylacetic Acid Derivatives and the Synthesis of Derived Penicillins." J.CHEM.SOC.PERKIN TRANS. I, vol. 6, 1984, pages 1227-1235, XP002064921

## Description

The present invention is concerned with a novel process for advantageously producing particular chloroacetylaminothiazoleacetic acid derivatives by the reaction of aminothiazoleacetic acid derivatives with a chloroacetylating agent on an industrial scale.

### (Background Art)

An aminothiazoleacetic acid derivative represented by the following formula (I) 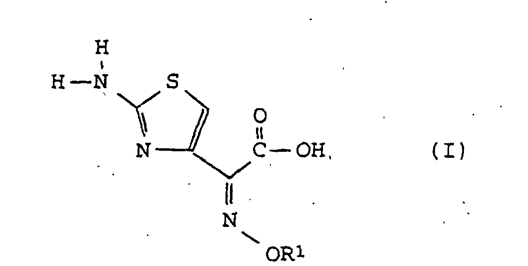 wherein R1 is a methyl protection group for a hydroxyl group,
is a compound which is useful as an intermediate for the preparation of pharmaceuticals, and serves an important compound forming a side chain of an antibiotic such as the one of the cephem type.

The above-mentioned compound is bonded to a ***β***-lactam compound such as 7-aminocephalosporanic acid derivative by the amide-forming reaction thereby to form a basic skeleton of an antibiotic.

In this case, the amino group of the aminothiazoleacetic acid derivative represented by the above formula (I) must be protected by some protection group in order to prevent the self-condensation. As a protection group for the amino group, there can be used many kinds of protection groups such as chloroacetyl group, triphenylmethyl group, alkoxycarbonyl group and the like groups. Among them, a
chloroacetylaminothiazoleacetic acid derivative expressed by the formula (II) is highly important with its amino group being protected by the chloroacetyl group from the standpoint of easily removing the protection group and economy, 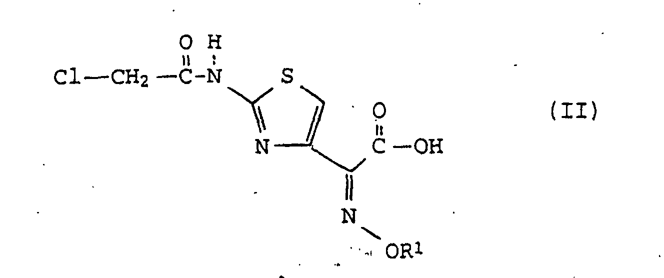 wherein R1 is a methyl protection group for the hydroxyl group.

So far, the chloroacetylaminothiazoleacetic acid derivatives have been prepared by:
(A) a process in which an aminothiazoleacetic acid ester is used as a starting material, which is reacted with a chloroacetylating agent to form an ester of chloroacetylaminothiazoleacetate, and, then, the ester group thereof is transformed into a carboxyl group (Japanese Laid-Open Patent Publications Nos. 125188/1977, 101393/1978, 103493/1978, 2809/1995); and
(B) a process in which an aminothiazoleacetic acid ester is hydrolyzed and is reacted with a chloroacetylating agent in the presence of a neutralizing agent such as a base like N-methylmorpholine, pyridine, trialkylamine, etc. (GB1580622).

According to the process (A), however, when the hydrolysis is executed for transforming the ester group into the carboxyl group, there is by-produced a compound in which a chlorine atom in the chloroacetyl group is substituted by other functional group, causing the yield of the desired compound to decrease. Besides, this by-product has properties resembling those of the desired compound, and cannot be easily removed from the object product despite of a refining operation such as crystallization, making it difficult to obtain the desired product in a highly pure form.

When the reduction with hydrogen is effected in order to transform the ester group into the carboxyl group, furthermore, a special reaction equipment is necessary requiring cumbersome operation such as removing the catalyst.

According to the process (B), on the other hand, the hydrolysis is effected before the chloroacetylation offering an advantage of suppressing the formation of the by-product that stems from the hydrolysis. The literature, however, does not at all contain a detailed description of the reaction. The present inventors, therefore, have conducted follow-up experiment and have found a problem as described below. That is, the by-product is formed in large amounts due to the reaction with the chloroacetylating agent, causing the conversion to decrease and making it difficult to carry out the process on an industrial scale.

It is therefore an object of the present invention to provide a process for effectively producing chloroacetylaminothiazoleacetic acid derivatives maintaining a high conversion and in a highly pure form.

The present inventors therefore have conducted a keen study concerning a process for production by using an aminothiazoleacetic acid derivative as a starting material without by-producing a hydrolyzed product, and have succeeded in synthesizing chloroacetylaminothiazoleacetic acid derivatives maintaining a high conversion and in a highly pure form by using an acid-trapping agent of the non-basic type which does not rely upon the neutralization reaction, but instead forms a complex compound with an acid or an addition reaction with an acid in the chloroacetylation in the above-mentioned process (B).

That is, the present invention provides a process for producing a chloroacetylaminothiazoleacetic acid derivative of the following formula (II) 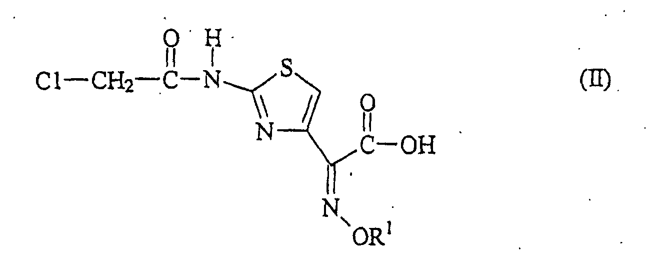 wherein R¹ is a methyl protection group which prevents a hydroxyl group from reacting with an agent for chloroacetylating the amino group, which comprises reacting in the presence of an acid-trapping agent an aminothiazoleacetic acid derivative of the following formula (I) 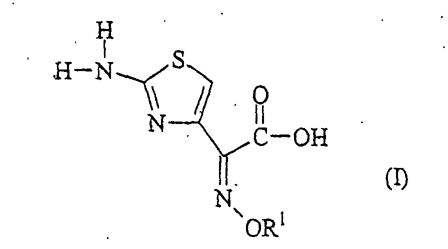 wherein R¹ is as defined above,
with the agent for chloroacetylating the amino group; the acid-trapping agent being N,N-dimethylformamide,
N,N-dimethylacetamide, N,N-diethylacetamide, N-methylpyrrolidone, N-ethylpyrrolidone, 4-pyrimidone, N-methylmaleimide, N-methylsuccinimide, 12-crown-4 ether, 15-crown-5 ether, 18-crown-6 ether, 1,2-dimethoxyethane, dimethyldiethylene glycol, dimethylpolyethylene glycol, dimethyltripropylene glycol, polyethylene glycol, ethylene oxide, propylene oxide, 1,2-epoxybutane, 1,2-epoxypentane, 1,2-epoxyhexane, 1,2-epoxyheptane, 1,2-epoxyoctane, 1,2-epoxynonane, 1,2-epoxydecane, 1,2-epoxydodecane, 1,2-epoxytetradecane, 1,2-epoxycyclopentane, 1,2-epoxycyclohexane, 1,4-epoxycyclohexane, 3,4-epoxy-1-butane, 3,4-epoxycyclohexylmethyl 3,4-epoxycyclohexanecarboxylate,
1,2-epoxy-9-decene, 3-epoxyethyl-7-oxabicylco[4,1,0] heptane, 1,2-epoxy-5-hexene, 1,2-epoxy-7-octene, exo-2,3-epoxynorbornane, exo-3,6-epoxy-1,2,3,6-tetrahydrophthalic acid anhydride, 1,2-epoxy-3-phenoxypropane, (2,3-epoxypropyl) benzene, 2,3-epoxypropyl furfuryl ether,
2,3-epoxypropyl methacrylate, 2,3-epoxypropyl 4-methoxyphenyl ether, N-(2,3-epoxypropyl) phthaLimide, 1,4-epoxy-1,2,3,4-tetrahydronaphthalene, 3,4-epoxytetrahydrothiophene-1,1-dioxide, epichlorohydrin, epibromohydrin, epifluorohydrin, styrene oxide, isobutylene, butadiene, isoprene, styrene, 2,3-dihydrofuran, 2,5-dihydrofuran, 4,5-dihydro-2-methylfuran, 3,4-dihydro-2H-pyran, 5,6-dihydro-2H-pyran-2- on, 3,4-dihydro-2-methoxy-2H-pyran or 3,4-dihydro-4-methyl-2H-pyran.

The compound capable of forming a complex preferably comprises an aprotic amide and/or a poly ether; and/or the compound capable of undergoing an addition reaction comprises one or more of an oxirane ring-containing compound, an aliphatic olefin compound, an aromatic olefin compound or an oxygen-containing aliphatic olefin compound.

In the present invention, R1 in the above-mentioned formula (I) is a protection group for the hydroxyl group. That is, if R1 were a hydrogen atom, a side reaction would take place with the chloroacetylating agent and, besides, the imino group is easily isomerized. Therefore, R1 must be a group other than hydrogen atom yet having the above-mentioned function, and is in fact a methyl group.

The compound represented by the above-mentioned formula (I) theoretically includes both syn- (Z) and anti-(E) isomers concerning the oxyimino group. In the present invention, the two isomers can be similarly used. By taking into consideration the pharmacological effects of pharmaceuticals incorporating the compound represented by the above-mentioned formula (I), however, it is desired to use the syn- (Z) isomer.

Though there is no particular limitation on the process for producing aminothiazoleacetic acid derivatives represented by the above-mentioned formula (I), it is desired to derive the derivatives from esters of aminothiazoleacetic acid derivatives represented by the following formula (III) 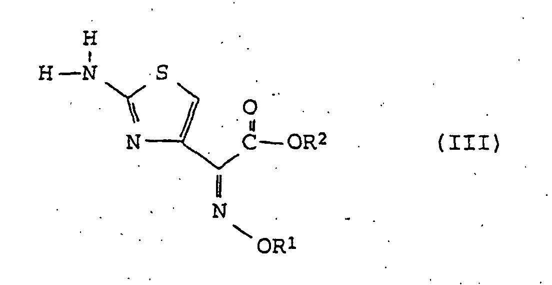 wherein R1 is as defined above, and R2 is an aralkyl or cyclic alkyl or straight chain alkyl protection group for the carboxyl group, in order to obtain the compounds in a highly pure form.

As the protection group for the carboxyl group denoted by R2 in the aminothiazoleacetic acid ester represented by the above-mentioned general formula (III), there can be used any group that is usually used as a protection group for the carboxyl group in the coupling reaction of amino acid or in the like reaction, without any limitation. Concrete examples of the above-mentioned group include straight or branched chain alkyl groups such as methyl group, ethyl group, n-propyl group, isopropyl group, n-butyl group, isobutyl group, and t-butyl group; aralkyl groups such as benzyl group and diphenylemethyl group; and cyclic alkyl groups such as cyclopentyl group and cyclohexyl group. Among them, it is desired to use alkyl groups with 1 to 4 carbon atoms, such as methyl group, ethyl group, isopropyl group and t-butyl group, or aralkyl groups such as benzyl group from the standpoint of easy refining after the protection group has been removed.

In general R² is preferably:
a straight chain or cyclic alkyl group, preferably having 8 or fewer carbon atoms, more preferably a straight alkyl group having 4 or fewer carbon atoms or a cyclic alkyl group having 5 or 6 carbon atoms; or
an aralkyl group preferably having 4 or fewer, more preferably 3 or fewer, rings and an alkyl group of 3 of fewer carbon atoms, more preferably a methyl group.

Examples of the ester of aminothiazoleacetic acid derivatives represented by the above-mentioned general formula (III) include:
methyl 2-(2-aminothiazole-4-yl)-2-methoxyiminoacetate; ethyl 2-(2-aminothiazole-4-yl)-2-methoxyiminoacetate; isopropyl 2-(2-aminothiazole-4-yl)-2-methoxyiminoacetate; t-butyl 2-(2-aminothiazole-4-yl)-2-methoxyiminoacetate; benzyl 2-(2-aminothiazole-4-yl)-2-methoxyiminoacetate; and cyclohexyl 2-(2-aminothiazole-4-yl)-2-methoxyiminoacetate.

A known method can be used without any particular limitation to derive the ester of aminothiazoleacetic acid derivatives into the aminothiazoleacetic acid derivatives represented by the above-mentioned formula (I) depending upon the kind of the protection group for the carboxyl group denoted by R2.

For example, when the protection group for the carboxyl group denoted by R2 can be easily hydrolyzed with a base or the like, there is employed a method which effects the hydrolysis in the presence of a basic aqueous solution followed by neutralization to obtain an aminothiazoleacetic acid derivatives.

In this case, any base can be used without particular limitation. Representative examples include alkali metal hydroxides such as lithium hydroxide, sodium hydroxide and potassium hydroxide; alkali metal carbonates such as lithium carbonate, sodium carbonate and calcium carbonate; alkaline earth metal carbonates such as magnesium carbonate and potassium carbonate; and alkali metal bicarbonates such as lithium hydrogencarbonate, sodium hydrogencarbonate and potassium hydrogencarbonate. Among them, sodium hydroxide and potassium hydroxide are particularly preferably used from the standpoint of their favorable reactivity.

When the base is used in a too small amount, the reaction requires an extended period of time. When the base is used in a too large amount, the acid must be used in large amounts for neutralization. It is therefore desired to use the base in an amount over a range of from 1 to 10 mols and, more preferably, from 1 to 5 mols per mol of the ester of an aminothiazoleacetic acid derivative represented by the above-mentioned formula (III).

The amount of the water used for the hydrolyzing reaction is preferably from 50 parts by weight to 1000 parts by weight per 100 parts by weight of the ester of the aminothiazoleacetic acid derivative by taking the reaction time and economy into consideration.

The hydrolyzing reaction may be conducted by using the water only as a solvent but, preferably, by using the water and the organic solvent which are mixed together. There is no particular limitation on the kind of the organic solvent. Concrete examples include aliphatic hydrocarbons such as pentane, hexane, heptane and trimethylpentane; cyclic aliphatic hydrocarbons such as cyclohexane; halogenated aliphatic hydrocarbons such as carbon tetrachloride, dichloromethane and trichloroethylene; aromatic hydrocarbons such as benzene, toluene and xylene; ethers such as diethyl ether, diisopropyl ether, butylmethyl ether, tetrahydrofurane and 1,4-dioxane; ketones such as acetone, methyl ethyl ketone and methylisobutyl ketone; esters such as ethyl acetate, t-butyl acetate, and n-propyl acetate; nitriles such as acetonitrile and propionitrile; alcohols such as methanol, ethanol, n-propyl alcohol, isopropyl alcohol, n-butyl alcohol, isobutyl alcohol and t-butyl alcohol; amides such as N,N-dimethylformamide and N,N-dimethylacetamide; sulfoxides such as dimethylsulfoxide; and amines such as triethylamine, N,N,N',N'-tetramethylethylenediamine and pyridine. Among them, there can be preferably used solvents having compatibility with the water, like ethers such as tetrahydrofurane and 1,4-dioxane; ketones such as acetone and methyl ethyl ketone; nitriles such as acetonitrile; alcohols such as methanol, ethanol, n-propyl alcohol, isopropyl alcohol, n-butyl alcohol, isobutyl alcohol and t-butyl alcohol.

These solvents may be used in a single kind or being mixed in two or more kinds together. There is no particular limitation on the amount of the solvent that is used. When the amount is too small, however, the stirring is affected. When the amount is too large, the production efficiency decreases per a batch. In general, therefore, it is desired to use the solvent in an amount of from 20 to 10000 parts by weight and, preferably, from 50 to 1000 parts by weight per 100 parts by weight of the ester of an aminothiazoleacetic acid derivative expressed by the above-mentioned formula (III).

There is no particular limitation on the temperature in the above-mentioned reaction. When the temperature is too low, however, an extended period of reaction time is required. When the temperature is too high, on the other hand, the aminothiazoleacetic acid derivatives are decomposed or isomerized. Usually, therefore, the reaction is conducted over a temperature range of from - 50°C to 100°C and, preferably, from 0°C to 80°C.

The reaction can be conducted under normal pressure, under an elevated pressure condition or under a reduced pressure condition. The time required for the reaction varies depending upon the reaction temperature and the kind of the solvent. Generally, however, the reaction time of from 0.1 to 60 hours is sufficient.

The thus obtained aminothiazoleacetic acid derivative salt is neutralized with an acid and is transformed into an aminothiazoleacetic acid derivative expressed by the above-mentioned formula (I). Representative examples of the acid used in this case include inorganic acids such as hydrochloric acid, nitric acid, sulfuric acid and phosphoric acid, and organic acids such as formic acid, acetic acid, citric acid, trifluoroacetic acid and p-toluenesulfonic acid. Among them, hydrochloric acid and sulfuric acid are favorably used from the standpoint of easy treatment after the reaction.

The acid is used for the neutralization reaction in an amount sufficient for neutralizing the base present in the reaction system. When the amount of the acid is too large, however, the formed product decomposes. It is therefore desired that the acid is added in such an amount that the pH of the reaction solution after the neutralization becomes from 0.1 to 6 and, preferably, from 1 to 4.

There is no particular limitation on the solvent used for the neutralization reaction. The solvent for the hydrolyzing reaction may be directly used or any other solvent may be used.

There is no particular limitation on the temperature for executing the neutralization reaction. When the temperature is too low, however, the solution is solidified. When the temperature is too high, on the other hand, the formed product is decomposed or isomerized. Usually, therefore, the neutralization reaction is executed at a temperature of from a solidifying point of the solution to 100°C and, preferably, from the solidifying point of the solution to 50°C.

Furthermore, when the group R2 of the ester of an aminothiazoleacetic acid derivative represented by the above-mentioned formula (III) can be easily removed by reduction, there is employed a method of obtaining the aminothiazoleacetic acid derivatives based on the reduction by using a reducing agent.

There is no particular limitation on the reducing method employed this time. If exemplified, however, there can be employed a method of reduction by using a hydrogen gas and by using palladium-carbon, Raney nickel or platinum oxide as a catalyst.

There is no particular limitation on the amount of using these reducing catalysts. In general, however, the reducing catalyst is used in an amount of from 0.01 to 100 parts by weight per 100 parts by weight of the ester of an aminothiazoleacetic acid derivative expressed by the above-mentioned formula (III). By taking economy and the like into consideration, furthermore, it is desired that the reducing catalyst is used in an amount of from 0.1 to 50 parts by weight.

Any known solvent can be used for the reaction without limitation provided it does not particularly take part in the catalytic reducing reaction. Examples of the solvent include aliphatic hydrocarbons such as pentane, hexane, heptane and trimethylpentane; cyclic aliphatic hydrocarbons such as cyclohexane; halogenated aliphatic hydrocarbons such as carbon tetrachloride, chloroform, dichloromethane, and trichloroethylene; aromatic hydrocarbons such as benzene, toluene and xylene; ethers such as diethyl ether, diisopropyl ether, butylmethyl ether, tetrahydrofurane and 1,4-dioxane; esters such as ethyl acetate, t-butyl acetate and n-propyl acetate; alcohols such as methanol, ethanol, n-propyl alcohol, isopropyl alcohol, n-butyl alcohol, isobutyl alcohol and t-butyl alcohol; amides such as N,N-dimethylformamide and N,N-dimethylacetamide; amines such as triethylamine, N,N,N',N'-tetramethylethylenediamine and pyridine, or a mixture solvent of these solvents and water. Among them, it is desired to use aprotic solvents like ethers such as tetrahydrofurane and 1,4-dioxane; esters such as ethyl acetate, t-butyl acetate and n-propyl acetate; and amides such as N,N-dimethylformamide and N,N-dimethylacetamide, since they make it possible to obtain the desired product in a highly pure form without accompanied by side reaction.

The reaction temperature may be selected by taking the catalyst, solvent and hydrogen pressure into consideration, and no particular limitation is imposed thereon. By taking the reaction rate into consideration, however, it is desired that the reaction temperature is from 0°C to 50°C.

In the above-mentioned reaction, the hydrogen pressure may be suitably selected depending upon the catalyst, solvent or the degree of progress of the reaction. Usually, however, the hydrogen pressure is selected to be from normal pressure to about 100 kg/cm² and, preferably, from normal pressure to about 50 kg/cm².

Any known method is used without limitation for separating the thus obtained aminothiazoleactic acid derivatives. Concretely speaking, there can be exemplified a filtering method such as natural filtration, pressurized filtration, reduced-pressure filtration or centrifugal filtration, solid-liquid separation method such as decantation or centrifugal separation, or an extraction method by using an organic solvent. When the aminothiazoleacetic acid derivatives are obtained by the reduction by using a catalyst, the catalyst is separated by the method of solid-liquid separation and, then, the aminothiazoleacetic acid derivatives are separated from the remaining components. The aminothiazoleacetic acid derivatives may be further separated and refined by the silica gel chromatography or may be crystallized by adding a bad solvent to further increase the purity.

The aminothiazoleacetic acid derivatives represented by the above-mentioned formula (I) of a high purity can be easily prepared by using the above-mentioned methods.

In the present invention, a known compound can be used without limitation as a chloroacetylating agent for the aminothiazole acetic acid derivatives. Concrete examples include acid anhydrides such as chloroacetic anhydride and anhydrous monochloroacetic acid; acid halides such as chloroacetyl chloride and chloroacetyl bromide; and chloroacetic acid. Among them, acid halides such as chloroacetyl chloride and chloroacetyl bromide can be preferably used from the standpoint of easy protection reaction and reaction rate.

Since the reaction is an equivalent reaction, the chloroacetylating agent may be used in an amount of not smaller than one mol per mol of the aminothiazoleacetic acid derivative which is the starting material. Use of the chloroacetylating agent in a too large amount is not economical and makes it difficult to remove an excess amount of the chloroacetylating agent. It is therefore desired that the chloroacetylating agent is used in an amount of from 1 mol to 3 mols per mol of the aminothiazoleacetic acid derivative.

The reaction proceeds very favorably owing to the presence of the acid-trapping agent. The acid-trapping agent in the reaction is not the one which removes acid by the neutralization reaction but is a compound that traps the acid, like a compound which forms a complex compound with the acid (hereinafter referred to as complex-forming type acid-trapping agent) or a compound capableof occurring an addition reaction with the acid (hereinafter referred to as addition reaction type acid-trapping agent).

The aminothiazoleacetic acid derivative expressed by the above-mentioned formula (I) has an amino group and a carboxyl group in the same molecule and forms an ion pair in a solvent. It has therefore been considered that the reaction with the amino group easily proceeds when the carboxyl group is neutralized with another base to form ion pairs among the molecules followed by the addition of the chloroacetylating agent, and, hence, the base has generally been used. However, the present inventors have repeated this method to only find the fact that the base gives rise to the occurrence of various problems contrary to the expectation.

On the other hand, among the acid-trapping agents used in the present invention, it has been known that the complex-forming type acid-trapping agent generally works to effectively chloroacetylate the ester of an aminothiazoleacetic acid derivative represented by the above-mentioned general formula (III). However, use of the complex-forming type acid-trapping agent for the chloroacetylation of amino groups in the aminothiazoleacetic acid derivatives having carboxyl groups in the molecules implies that the isomerization reaction of the oximino groups may be promoted in the presence of the acid-trapping agent. In fact, nobody has ever used the addition reaction type acid-trapping agent for the chloroacetylation reaction.

The present inventors have discovered for the first time an astonishing effect in that use of the acid-trapping agent makes it possible to obtain chloroacetylaminothiazoleacetic acid derivatives in a highly pure form maintaining a very high conversion compared with the conventional method of using a neutralizing agent.

The acid-trapping agent used in the present invention may be a complex-forming type acid-trapping agent, namely N,N-dimethylformamide, N,N-dimethylacetamide, N,N-diethylacetamide, N-methylpyrrolidone, N-ethylpyrrolidone, 4-pyrimidone, N-methylmaleimide, N-methylsuccinimide, 12-crown-4 ether, 15-crown-5 ether, 18-crown-6 ether, 1,2-dimethoxyethane, dimethylidiethylene glycol, dimethylpolyethylene glycol, dimethyltripropylene glycol or polyethylene glycol. Alternatively, an addition reaction type acid-trapping agent may be used, namely ethylene oxide, propylene oxide, 1,2-epoxybutane, 1,2-epoxypeptane, 1,2-epoxyhexane, 1,2-epoxyheptane, 1,2-epoxyoctane, 1,2-epoxynonane, 1,2-epoxydecane, 1,2-epoxydodecane, 1,2-epoxytetradecane, 1,2-epoxyhexadecane, 1,2-epoxyoctadecane, 1,2-epoxycyclopentane, 1,2-epoxycyclohexane, 1,4-epoxycyclohexane, 3,4-epoxy-1-butene, 3,4-epoxycyclohexylmethyl 3,4-epoxycyclohexanecarboxylate, 1,2-epoxy-9-decene, 3-epoxyethyl-7-oxabicyclo[4,1,0] heptane, 1,2-epoxy-5-hexene, 1,2-epoxy-7-octene, exo-2,3-epoxynorbornane, exo-3,6-epoxy-1,2,3,6-tetrahydrophthalic acid anhydride, 1,2-epoxy-3-phenoxypropane, (2,3-epoxypropyl) benzene, 2,3-epoxypropyl furfuryl ether, 2,3-epoxypropyl methacrylate, 2,3-epoxypropyl 4-methoxyphenyl ether, N-(2,3-epoxypropyl)phthalimide, 1,4-epoxy-1,2,3,4-tetrahydronaphthalene, 3,4-epoxytetrahydrothiophene-1,1-dioxide, epichlorohydrin, epibromohydrin, epifluorohydrin, styrene oxide, isobutylene, butadiene, isoprene, styrene, 2,3-dihydrofuran, 2,5-dihydrofuran, 4,5-dihydro-2-methylfuran, 3,4-dihydro-2H-pyran, 5,6-dihydro-2H-pyran-2- one, 3,4-dihydro-2-methoxy-2H-pyran or 3,4-dihydro-4-methyl-2H-pyran.

Among the above-mentioned complex-forming type acid-trapping agents, there are preferably used amides such as N,N-dimethylformamide, N,N-dimethylacetamide, N,N-diethylacetamide, N-methylpyrrolidone, N-ethylpyrrolidone, 4-pyrimidone, N-methylmaleimide and N-methylsuccinimide, since they make it possible to obtain desired products maintaining high selectivity permitting side reactions to take place little.

Among the above-mentioned addition reaction type acid-trapping agents, there are preferably used oxide compounds such as ethylene oxide, propylene oxide, 1,2-epoxybutane, 1,2-epoxypentane, 1,2-epoxyhexane, 1,2-epoxyheptane, 1,2-epoxyoctane, 1,2-epoxynonane, 1,2-epoxydecane, 1,2-epoxycyclopentane, 1,2-epoxycyclohexane, 3,4-epoxy-1-butene, 1,2-epoxy-9-decene, 1,2-epoxy-5-hexene, 1,2-epoxy-7-octene, 1,2-epoxy-3-phenoxypropane, 2,3-epoxypropyl methacrylate, epichlorohydrin, epibromohydrin, epifluorohydrin and styrene oxide; and oxygen-containing aliphatic olefin compounds such as 2,3-dihydrofuran, 4,5-dihydro-2-methylfuran, 3,4-dihydro-2H-pyran, 3,4-dihydro-2-methoxy-2H-pyran and 3,4-dihydro-4-methyl-2H-pyran, since they make it possible to obtain desired products maintaining high selectivity permitting side reactions to take place little.

Among the acid-trapping agents used in the present invention, use of the addition reaction type acid-trapping agent and, particularly, use of the oxide compounds is very advantageous from an industrial point of view since they are soluble in an organic solvent, enabling the chloroacetylaminothiazoleacetic acid derivative which is the object product to be directly separated from the solvent by crystallization without using water in the step of separation and refining, without requiring the step of extraction with water that is necessary when the complex-forming type acid-trapping agent is used.

In the present invention, there is no particular limitation on the amount of using the acid-trapping agent so far as the function of the invention is exhibited. That is, when the amount of use is too small, little effect is exhibited for suppressing the isomerization. Generally, therefore, the acid-trapping agent is used in an amount of not smaller than 0.5 equivalent and, preferably, not smaller than 1.0 equivalent with respect to the acid formed in the reaction system.

There is no particular limitation on the upper limit of the amount of the acid-trapping agents since the acid-trapping agents themselves can be used at least as part of the solvent.

The acid-trapping agents may be used in a single kind or, as required, in two or more kinds being mixed together.

When the acid-trapping agents themselves are used at least as part of the solvent for the reaction, there are preferably used those that remain liquid at normal temperature. Concrete examples include, in the case of the complex-forming type acid-trapping agents, amides such as N,N-dimethylformamide, N,N-dimethylacetamide, N,N-diethylacetamide, N-methylpyrrolidone, and N-ethylpyrrolidone, and include, in the case of the addition reaction type acid-trapping agents, oxide compounds such as propylene oxide, 1,2-epoxybutane, 1,2-epoxypentane, 1,2-epoxyhexane, 1,2-epoxyheptane, 1,2-epoxyoctane, 1,2-epoxynonane, 1,2-epoxydecane, 1,2-epoxycyclopentane, 1,2-epoxycyclohexane, 3,4-epoxy-1-butene, epichlorohydrin and styrene oxide; aliphatic olefin compounds such as isoprene; aromatic olefin compounds such as styrene; and oxygen-containing aliphatic olefin compounds such as 2,3-dihydrofuran, 4,5-dihydro-2-methylfuran, 3,4-dihydro-2H-pyran, 3,4-dihydro-2-methoxy-2H-pyran, and 3,4-dihydro-4-methyl-2H-pyran. Among them, it is preferred to use amides such as N,N-dimethylformamide, N,N-dimethylacetamide, N,N-diethylacetamide, N-methylpyrrolidone and N-ethylpyrrolidone, from the standpoint of high yields and high reaction rates.

When another solvent is to be used for the chloroacetylating reaction of the present invention, any known organic solvent can be used without limitation. Examples of such a solvent include aliphatic hydrocarbons such as pentane, hexane, heptane and trimethylpentane; cyclic aliphatic hydrocarbons such as cyclohexane; halogenated aliphatic hydrocarbons such as carbon tetrachloride, chloroform, dichloromethane and trichloroethylene; aromatic hydrocarbons such as benzene, toluene and xylene; ethers such as diethyl ether, diisopropyl ether, butylmethyl ether, tetrahydrofuran and 1,4-dioxane; ketones such as acetone, methyl ethyl ketone and methylisobutyl ketone; esters such as ethyl acetate, t-butyl acetate and n-propyl acetate; carbonates such as dimethyl carbonate; nitriles such as acetonitrile and propionitrile; alcohols such as t-butyl alcohol and t-amyl alcohol; and dimethyl sulfoxide.

Among them, there are preferably used ethers such as tetrahydrofuran and 1,4-dioxane; ketones such as acetone and methyl ethyl ketone; and esters such as ethyl acetate and n-propyl acetate from the standpoint of suppressing the isomerization.

The above-mentioned solvents may be used in a single kind or in two or more kinds being mixed together.

In the chloroacetylation reaction of the present invention, there is no particular limitation on the concentration of the aminothiazoleacetic acid derivatives represented by the general formula (I) in the reaction system. When the concentration is too high, however, the stirring is affected. When the concentration is too low, on the other hand, the production efficiency decreases per a batch. In general, therefore, it is desired that the concentration is adjusted to be from 0.1 to 80% by weight and, preferably, from 1 to 70% by weight.

The temperature of the chloroacetylation reaction varies depending upon the kind of the aminothiazoleacetic acid derivative that is used, presence and kind of the acid-trapping agent, and cannot be exclusively determined. When the temperature is too low, however, the reaction rate becomes small. When the temperature is too high, on the other hand, the imino group in the aminothiazoleacetic acid derivative is isomerized, or a chloroacetylated product (hereinafter referred to as chloroacetyldithiazoleacetic acid derivative) of a dimer (hereinafter referred to as dithiazoleacetic acid derivative) of the aminothiazoleacetic acid derivative is by-produced as expressed by the following formula (IV) 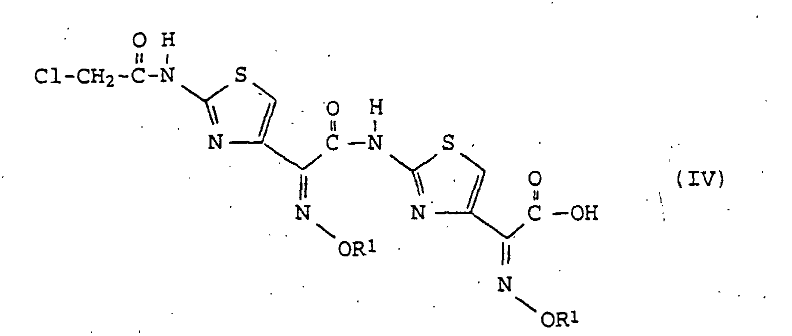 wherein R1 is a methyl protection group for the hydroxyl group.

Usually, therefore, the temperature is selected to lie from the solidifying point of the reaction system to 100°C. When olefin compounds or oxide compounds that strongly interact with the acid are used as acid-trapping agents, it is desired that the reaction is carried out at a temperature of from -30°C to 80°C. When amides or ethers having a weak interaction are used, it is desired that the reaction is carried out at a temperature of solidifying point of the reaction system to 20°C.

As for the chloroacetyldithiazoleacetic acid derivatves represented by the above-mentioned formula (IV), impurities are contained in the product irrespective of the temperature when dithiazoleacetic acid derivatives are contained in the aminothiazoleacetic acid derivatives that are the starting materials. However, the dithiazoleacetic acid derivatives are transformed into aminothiazoleacetic acid derivatives with an increase in the temperature. It is therefore desired that the temperature is maintained within the above-mentioned range.

The above-mentioned reaction is carried out under normal pressure, elevated pressure or reduced pressure. The time required for the reaction varies depending upon the reaction temperature, kind of the solvent and kind of the acid-trapping agent. Usually, however, the reaction time is suitably selected to be from 0.1 to 100 hours.

The reaction can be carried out in an open air. Here, however, the chloroacetylating agent used for the reaction easily reacts with the water. It is therefore desired to carry out the reaction in an apparatus equipped with a drier tube or in an inert gas atmosphere such as of nitrogen or argon.

Thus, the chloroacetylaminothiazoleacetic acid derivatives expressed by the above-mentioned formula (II) are formed.

In the present invention, the chloroacetylaminothiazoleacetic acid derivatives expressed by the above-mentioned formula (II) are separated and refined by a known separation method depending upon the kinds of the acid-trapping agent and the solvent.

Described below is a representative method of separation and refining. When amides in the complex-forming type acid-trapping agent are used as acid-trapping agents, a mixture of the complex and the product may precipitate from the reaction system. It is therefore necessary to separate the complex only from the product. When an organic solvent that dissolves in the water is used, therefore, the solvent is distilled off or is not distilled off but is mixed with the water. Then, the crystals that are precipitating are separated by a known solid-liquid separation method. Or, the organic solvent that does not dissolve in the water is added thereto to extract the crystals and, then, the solvent is distilled off. Or, depending upon the cases, a solvent (hereinafter simply referred to as bad solvent) that little dissolves the chloroacetylaminothiazoleacetic acid derivatives expressed by the above-mentioned formula (II) is added thereto to separate and refine the chloroacetylaminothiazoleacetic acid derivatives represented by the above-mentioned formula (II).

When an organic solvent that does not dissolve in the water is used, the organic solvent is mixed into the water to remove the remaining chloroacetylating agent, complex and acid-trapping agent. Thereafter, the chloroacetylaminothiazoleacetic acid derivatives of the above-mentioned formula (II) are separated and refined by the same method as the one used for the above-mentioned operation for the extraction. When the chloroacetylaminothiazoleacetic acid derivatives are to be extracted by using the organic solvent, a batchwise extraction method is generally employed. However, the chloroacetylaminothiazoleacetic acid derivatives expressed by the above-mentioned formula (II) formed by the reaction dissolves little in the organic solvent. Therefore, the batchwise extraction method often requires the organic solvent in large amounts. In such a case, it is desired to employ a continuous extraction method. Any known continuous extraction method can be used without limitation. Concrete examples include counter-current extraction methods by using static towers such as filler tower, seeptray tower; mixer-settlers; stirrer-type towers such as Scheibel tower and Mixco tower; pulsation type towers such as filler-type pulse tower and seep-tray-type pulse tower; rotating disk columns; and reciprocating plate-type towers such as carl-column. These methods make it possible to obtain the traditionally known effect of decreasing the amounts of the washing water and the waste water, as well as to obtain the effect of enhancing the purity of the object product of the invention.

When the addition reaction type acid-trapping agent is used, on the other hand, there can be employed quite a different separation method. That is, the addition reaction-type acid-trapping agent reacts with the acid to form a compound that dissolves in an organic solvent. When a bad solvent is used for the reaction, therefore, the product precipitates from the reaction system and is separated by the solid-liquid separation. When a solvent other than the bad solvent is used, the solvent is distilled off or a bad solvent is added thereto to precipitate the product which is, then, separated and refined by solid-liquid separation.

By using the addition reaction type acid-trapping agent, as described above, the process is carried out from the reaction up to the separation and refining without at all using the water. Therefore, there does not at all occur a side reaction in which chlorine atoms of the chloroacetylaminothiazoleacetic acid derivatives which are the products are substituted. Besides, the step of extraction can be omitted, the apparatus for production is constructed in a small size and in a simple structure offering great advantage.

Among the complex-forming type acid-trapping agents, furthermore, when ethers are used, the complex is not precipitated from the reaction system unlike when amides are used and, hence, the operation is carried out in the same manner as that of when the addition reaction type acid-trapping agents are used. When ethers are used, however, hydrogen chloride exists in an unreacted form like that of when the addition reaction type acid-trapping agents are used. In isolating the product, therefore, the product must be washed with a bad solvent to a sufficient degree so that no hydrogen chloride remains in the product.

The bad solvent used in the present invention may differ depending upon the kind of the chloroacetylaminothiazoleacetic acid derivative represented by the above-mentioned formula (I). However, any bad solvent may be used without limitation provided the compound little dissolves therein.

Concrete examples include aliphatic halogenated hydrocarbons such as dichloromethane, chloroform and carbon tetrachloride; aliphatic hydrocarbons such as hexane, heptane and cyclohexane; aromatic hydrocarbons such as toluene and xylene; carbonates such as dimethylcarbonate; esters such as ethyl acetate, n-propyl acetate, isopropyl acetate, n-butyl acetate, isobutyl acetate and t-butyl acetate; alcohols such as isopropyl alcohol, t-butyl alcohol and isoamyl alcohol; ethers such as diethyl ether and diisopropyl ether; and ketones such as acetone and methyl ethyl ketone.

Among these solvents, it is desired to use polar solvents like aliphatic halogenated hydrocarbons such as dichloromethane and chloroform; carbonates such as dimethyl carbonate; esters such as ethyl acetate, n-propyl acetate, isopropyl acetate, n-butyl acetate, isobutyl acetate, and t-butyl acetate; alcohols such as isopropyl alcohol, t-butyl alcohol and isoamyl alcohol; ethers such as diethyl ether and diisopropyl ether; and ketones such as acetone and methyl ethyl ketone, that permit the chloroacetyldithiazoleacetic acid derivatives expressed by the above-mentioned general formula (IV) by-produced by the reaction to be highly dissolved therein, and that exhibit a large difference in the solubility for the synproduct (Z-product) and anti-product (E-product) of the chloroacetylaminothiazoleacetic acid derivatives represented by the above-mentioned general formula (II).

The ratio of mixing a bad solvent and a solvent other than the bad solvent cannot be exclusively determined since the solubility differs depending upon the kind of the chloroacetylaminothiazoleacetic acid derivative expressed by the above-mentioned formula (II) or upon the solvent, or the solubility of the solvent differs depending upon the impurities. Usually, however, mixture solvents are separately prepared by using the solvent other than the bad solvent and the bad solvent at different ratios, and the solubilities of the chloroacetylaminothiazoleacetic acid derivatives 5 represented by the above-mentioned formula (II) and of the impurities in the mixture solvents are measured, and a suitable mixing ratio is determined.

These methods make it possible to obtain chloroacetylaminothiazoleacetic acid derivatives having sufficiently high purities. In order to further increase the purity, furthermore, they may be separated and refined by the silica gel chromatography or may be recrystallized.

By reacting the aminothiazoleacetic acid derivatives represented by the above-mentioned formula (I) with the chloroacetylating agent as described above, it is made possible to advantageously produce the chloroacetylaminothiazoleacetic acid derivatives on an industrial scale.

According to the present invention, the 0 aminothiazoleacetic acid derivatives represented by the above-mentioned formula (I) are used as starting materials and are reacted with the chloroacetylating agent in the presence of an acid-trapping agent, in order to obtain the chloroacetylaminothiazoleacetic acid derivatives represented by the above-mentioned formula (II) in a highly pure form maintaining high yields. Therefore, the present invention offers a very great industrial merit.

The invention will now be concretely described by way of Examples. In the following Tables, the E-body represents isomers of the starting materials and of the object products, and the dimer represents the dithiazoleacetic acid derivatives concerning the starting materials and represents the chloroacetyldithiazoleacetic acid derivatives concerning the object products.

### (Example 1)

60 Milliliters of an N,N-dimethylacetamide was introduced into a 200-ml eggplant-type flask equipped with a dryer tube filled with calcium chloride, and was cooled down to -15°C, to which was then added 20.1 g (0.1 mol) of a 2-(2-aminothiazole-4-yl)-2-(Z)-methoxyiminoacetic acid. After the liquid temperature became not higher than -5°C, 13.6 g (0.12 mols) of a chloroacetyl chloride was dropwisely added thereto while maintaining the temperature of the reaction solution to be not higher than 0°C.

The 2-(2-aminothiazole-4-yl)-2-(Z)-methoxyiminoacetic acid used as the starting material was analyzed by HPLC, and it was found that its purity was 99.00% containing impurities, i.e., 2-(2-aminothiazole-4-yl)-2-(E)-methoxyiminoacetic acid in an amount of 0.09% and dithiazoleacetic acid derivative in an amount of 0.10%.

Thereafter, the reaction was conducted for 3 hours while maintaining the liquid temperature at not higher than 0°C. After the reaction, the reaction solution was added to 200 ml of the ion-exchanged water and was extracted twice each with 200 ml of ethyl acetate. The extracts were combined together, washed four times each with 100 ml of the ion-exchanged water and, then, ethyl acetate was distilled off under a reduced pressure, followed by the addition of 100 ml of hexane to filtrate the precipitated crystals.

The obtained crystals were dried under a reduced pressure to obtain 23.6 g of a 2-(2-chloroacetylaminothiazole-4-yl)-2-(Z)-methoxyiminoacetic acid. The yield was 85.1%. The obtained substance was analyzed by a high-speed liquid chromatography (hereinafter abbreviated as HPLC) and it was found that the purity was 99.80% containing impurities, i.e., 2-(2-chloroacetylaminothiazole-4-yl)-2-(E)-methoxyiminoacetic acid in an amount of 0.05% and chloroacetyldithiazoleacetic acid derivative in an amount of 0.07%. Furthermore, the 2-(2-aminothiazole-4-yl)-2-(Z)-methoxyiminoacetic acid was contained in an amount of 0.02%.

### (Example 2)

200 Milliliters of ethanol was added to a solution obtained by suspending 45.9 g (0.2 mols) of an ester of an ethyl 2-(2-aminothiazole-4-yl)-2-(Z)-methoxyiminoacetate in 250 ml of a 1N sodium hydroxide aqueous solution, followed by stirring at room temperature for 15 hours. The obtained reaction solution was adjusted for its pH to be 7.0 with 10% hydrochloric acid, and ethanol was distilled off under a reduced pressure. The water layer was washed with ethyl acetate, adjusted for its pH to 2.8 with 10% of hydrochloric acid, and was stirred while being cooled with ice, and crystals were precipitated.

The crystals were separated by filtration, washed with acetone, and were recrystallized from ethanol to obtain a 2-(2-aminothiazole-4-yl)-2-(Z)-methoxyiminoacetic acid in an amount of 22.9 g (yield, 57.0%). The obtained product was analyzed by HPLC and it was found that the purity was 98.20% containing impurities, i.e., (2-aminothiazole-4-yl)-(E)-methoxyiminoacetic acid in an amount of 0.23% and dithiazoleacetic acid derivative in an amount of 0.20%.

By using 20.1 g (0.1 mol) of the 2-(2-aminothiazole-4-yl)-2-(Z)-methoxyiminoacetic acid, the procedure same as that of Example 1 was repeated to obtain 23.5 g of the 2-(2-chloroacetylaminothiazole-4-yl)-2-(Z)-methoxyiminoacetic acid. The yield was 84.7%. The obtained product was analyzed by HPLC and it was found that the purity was 98.60% containing impurities, i.e., 2-(2-chloroacetylaminothiazole-4-yl)-2-(E)-methoxyiminoacetic acid in an amount of 0.11% and chloroacetyldithiazoleacetic acid derivative in an amount of 0.13%.

### (Example 3)

The procedure was repeated in the same manner as in example 2 but using an ester of a methyl 2-(2-aminothiazole-4-yl)-2-(Z)-methoxyiminoacetate as a starting material to obtain 23.6 g of the 2-(2-chloroacetylaminothiazole-4-yl)-2-(Z)-methoxyiminoacetic acid. The purity was 98.90% containing impurities, i.e., 2-(2-chloroacetylaminothiazole-4-yl)-2-(E)-methoxyiminoacetic acid in an amount of 0.10% and chloroacetyldithiazoleacetic acid derivative in an amount of 0.02%. Furthermore, a 2-(2-aminothiazole-4-yl)-2-(Z)-methoxyiminoacetic acid obtained during the reaction was analyzed by HPLC, and it was found that the purity was 98.83% containing impurities, i.e., 2-(2-aminothiazole-4-yl)-2-(E)-methoxyiminoacetic acid in an amount of 0.11% and dithiazoleacetic acid derivative in an amount of 0.03%.

### (Example 4)

58.3 Grams (0.2 mols) of an ester of a benzyl 2-(2-aminothiazole-4-yl)-2-(Z)-methoxyiminoacetate was dissolved in 3 liters of ethyl acetate, followed by the addition of 14.5 g of a 10% Pd-C. The mixture was reacted with stirring under a hydrogen pressure of 20 kg/cm² at room temperature for 4 hours. After the reaction, 3 liters of methanol was added to the reaction solution and the catalyst was removed by filtration. The residue obtained by condensing the filtrate under a reduced pressure was recrystallized with 600 ml of water-containing methanol to obtain a 2-(2-aminothiazole-4-yl)-2-(Z)-methoxyiminoacetic acid in an amount of 24.2 g (yield 60.1%).

The obtained product was analyzed by HPLC, and it was found that the purity was 97.31% containing a 2-(2-aminothiazole-4-yl)-2-(E)-methoxyiminoacetic acid in an amount of 0.55%. No dithiazoleacetic acid derivative was detected. By using 20.1 Grams (0.1 mol) of the 2-(2-aminothiazole-4-yl)-2-(Z)-methoxyiminoacetic acid, the procedure was carried out in the same manner as in Example 1 to obtain 23.3 g of the 2-(2-chloroacetylaminothiazole-4-yl)-2-(Z)-methoxyiminoacetic acid. The yield was 84.1%. The obtained product was analyzed by HPLC and it was found that the purity was 98.24% containing 2-(2-chloroacetylaminothiazole-4-yl)-2- (E) -methoxyiminoacetic acid in an amount of 0.27% and chloroacetyldithiazole derivative in an amount of 0.02%.

### (Examples 5 and 6)

The procedure was repeated in the same manner as in Example 1 but using the compounds shown in Table 1 as chloroacetylating agents. The results were as shown in Table 1.

**Table 1**

| Ex. | Chloroacetylating agent | Object product | Purity of object product | Yield of object product |
|---|---|---|---|---|
| 5 | chloroacetic acid anhydride | 2-(2-chloroacetylaminothiazole-4-yl)-2-(z)-methoxy-iminoacetic acid | 99.4% containing 0.05% of E-body and 0.09% of dimer | 84.2% |
| | | | | |
| 6 | chloroacetyl bromide | 2-(2-chloroacetylaminothiazole-4-yl)-2-(z)-methoxy-iminoacetic acid | 99.0% containing 0.05% of E-body and 0.15% of dimer | 84.9% |

### (Examples 7 to 9)

The procedure was repeated in the same manner as in Example 1 but using the solvents shown in Table 2 as the reaction solvents for periods of reaction time shown in Table 2. The results were as shown in Table 2.

**Table 2**

| Ex. | Reaction solvent | Reaction time | Purity of object product | Yield of object product |
|---|---|---|---|---|
| 7 | N,N-dimethylform-amide | 12 hours | 98.1% containing 0.49% of E-body and 0.15% of dimer | 84.1% |
| | | | | |
| 8 | N,N-diethylacet-amide | 5 hours | 99.8% containing 0.10% of E-body and 0.11% of dimer | 85.0% |
| | | | | |
| 9 | N-methyl-pyrrolidone | 12 hours | 98.3% containing 0.30% of E-body and 0.10% of dimer | 84.2% |

### (Example 10)

1.74 Kilograms (8.65 mols) of the same 2-(2-aminothiazole-4-yl)-2-(Z)-methoxyiminoacetic acid as the one used in Example 1 and 5.2 liters of an N,N-dimethylacetamide were added to a 50-liter reaction oven made of GL in a nitrogen atmosphere, and were cooled down to -12°C. After cooling, 1.17 kg (10.35 mol) of a chloroacetyl chloride was slowly and dropwisely added over a period of one hour so that the internal temperature became not higher than -10°C.

After the dropwise addition has been finished, the reaction was conducted at an inner temperature of not higher than -8°C for 5 hours. After the reaction has been finished, 5 liters of water was added thereto, and the liquid-liquid extraction was effected with 20 liters of ethyl acetate (flow rate of the extracting agent of 4 liters/hour) and 30 liters of water (feeding rate of 7 liters/hour) by using a carlcolumn counter-current extraction device (reciprocal plate type) having five theoretical number of stages.

The obtained ethyl acetate solution was condensed under a reduced pressure, 8 liters of methylene chloride was added thereto, and the mixture was stirred at 0°C for one hour. The crystals were removed by filtration, washed with 4 liters of methylene chloride, and were dried under a reduced pressure to obtain 2.16 kg of the 2-(2-chloroacetylaminothiazole-4-yl) -2- (Z)-methoxyiminoacetic acid. The yield was 89.9%.

The obtained product was analyzed by HPLC, and it was found that the purity was 99.81% containing impurities, i.e., 2-(2-chlbroacetylaminothiazole-4-yl)-2-(E)-methoxyiminoacetic acid in an amount of 0.04%. The 2-(2-aminothiazole-4-yl)-2-(Z)-methoxyiminoacetic acid was contained in an amount smaller than a detectable limit.

### (Example 11)

210.1 Grams (1.04 mols) of the same 2-(2-aminothiazole-4-yl)-2-(Z)-methoxyiminoacetic acid as the one used in Example 1, 300 ml of a tetrahydrofuran and 145.6 g (2.51 mols) of a propylene oxide were added into a 1-liter four neck distillation flask equipped with a stirrer and a dryer tube filled with calcium chloride, and were cooled down to 5°C. After cooling, 235.9 g (2.09 mols) of the chloroacetyl chloride was slowly and dropwisely added over a period of one hour so that the inner temperature was not higher than 12°C. After the dropwise addition has been finished, the inner temperature was raised up to 20°C to conduct the reaction for 20 hours. The temperature was then raised to 50°C and the reaction was further conducted for another 6 hours.

After the reaction has been finished, 130 ml of the tetrahydrofuran was distilled off under a reduced pressure. To the obtained slurry solution was added 350 ml of methylene chloride, followed by stirring to a sufficient degree. The crystals were separated by filtration by suction, and were washed twice with 170 ml of methylene chloride and were dried under a reduced pressure to obtain 231.9 g of the 2-(2-chloroacetylaminothiazole-4-yl)-2-(Z)-methoxyiminoacetic acid. The yield was 80.0%.

The product was analyzed by HPLC and it was found that the purity was 99.74% containing impurities, i.e., 2-(2-chloroacetylaminothiazole-4-yl)-2-(E)-methoxyiminoacetic acid in an amount of 0.02% and chloroacetyldithiazoleacetic acid derivative in an amount of 0.02%.

### (Examples 12 to 14)

The procedure was repeated in the same manner as in Example 11 but using a tetrahydrofuran as a reaction solvent, decreasing the reaction scale down to one-fifth, adding acid-trapping agents shown in Table 3, and conducting the reaction after the addition of the chloroacetyl chloride under the conditions of reaction temperatures and reaction times shown in Table 3. The results were as shown in Table 3.

**Table 3**

| Ex. | Acid-trapping agent and amount | Reaction temp. (°C) | Reaction time (hr) | Purity of object product | Yield of object product |
|---|---|---|---|---|---|
| 12 | styrene oxide/ 5.0 eq. | 20 50 | 20 7 | 98.0% containing 1.00% of E-body and 0.03% of dimer | 75.4% |
| | | | | | |
| 13 | 3,4-dihydro-2H-pyran/15 eq. | 30 50 | 30 10 | 94.8% containing 1.00% of E-body and 0.05% of dimer | 64.8% |
| | | | | | |
| 14 | 1,2-epoxybutane/ 3.0 eq. | 20 50 | 20 6 | 98.1% containing 1.38% of E-body and 0.02% of dimer | 78.8% |

### (Examples 15 and 16)

The procedure was repeated in the same manner as in Example 1 but adding 60 ml of a tetrahydrofuran instead of 60 ml of the N,N-dimethylacetamide, increasing the amount of the chloroacetyl chloride to 0.2 mols, and adding the acid-trapping agents shown in Table 4.

**Table 4**

| Ex. | Acid-trapping agent and amount | Reaction temp. (°C) | Reaction time (hr) | Purity of object product | Yield of object product |
|---|---|---|---|---|---|
| 15 | N,N-dimethyl-acetamide/4 eq. | 0 | 10 | 98.0% containing 0.33% of E-body and 0.09% of dimer | 83.8% |
| | | | | | |
| 16 | N,N-dimethyl-formamide/4 eq. | 0 | 10 | 97.7% containing 1.00% of E-body and 0.11% of dimer | 81.6% |

### (Examples 17 an 18)

The procedure was repeated in the same manner as in Example 11 but reducing the reaction scale down to one-fifth, using organic solvents shown in Table 5, and conducting the reaction under the conditions of reaction temperatures and reaction times shown in Table 5. The results were as shown in Table 5.

**Table 5**

| Ex. | Kind of organic solvent temp. | Reaction (°C) | Reaction time (hr) | Purity of object product | Yield of object product |
|---|---|---|---|---|---|
| 17 | acetone | 5 | 21 | 98.0% containing 1.33% of E-body and 0.05% of dimer | 80.8% |
| | | | | | |
| 18 | ethyl acetate | 5 | 5 | 97.7% containing 1.04% of E-body and 0.02% of dimer | 81.6% |

### (Example 19)

60.3 Grams (0.3 mols) of the same 2-(2-aminothiazole-4-yl)-2-(z)-methoxyiminoacetic acid as the one used in Example 1, 90 ml of a tetrahydrofuran and 41.8 g (0.72 mols) of a propylene oxide were added into a 300-milliliter four neck distillation flask equipped with a stirrer and a dryer tube filled with calcium chloride, and were cooled down to 0°C. After cooling, 67.8 g (0.6 mols) of the chloroacetyl chloride was slowly and dropwisely added over a period of one hour so that the inner temperature was not higher than 12°C. After the dropwise addition has been finished, the inner temperature was raised up to 25°C to conduct the reaction for 16 hours. The temperature was then raised to 50°C and the reaction was further conducted for another 3 hours. After the reaction has been finished, 30 ml of the tetrahydrofuran was distilled off under a reduced pressure.

To the obtained slurry solution was added 100 ml of isopropyl alcohol, followed by stirring at 0°C for one hour. The crystals were separated by using a small centrifugal filtering machine, and were washed twice with 100 ml of isopropyl alcohol and were dried under a reduced pressure to obtain 59.9 g of the 2-(2-chloroacetylaminothiazole-4-yl)-2-(Z)-methoxyiminoacetic acid. The yield was 71.9%.

The product was analyzed by HPLC and it was found that the purity was 99.63% containing impurities, i.e., 2-(2-chloroacetylaminothiazole-4-yl)-2-(E)-methoxyiminoacetic acid in an amount of 0.01% and chloroacetyldithiazoleacetic acid derivative in an amount of 0.01%.

### (Example 20)

60.3 Grams (0.3 mols) of the same 2-(2-aminothiazole-4-yl)-2-(Z)-methoxyiminoacetic acid as the one used in Example 1, 90 ml of a tetrahydrofuran and 41.8 g (0.72 mols) of a propylene oxide were added into a 300-milliliter four neck distillation flask equipped with a stirrer and a dryer tube filled with calcium chloride, and were cooled down to 10°C. After cooling, 67.8 g (0.6 mols) of the chloroacetyl chloride was slowly and dropwisely added over a period of 2 hours so that the inner temperature was from 20°C to not higher than 25°C. After the dropwise addition has been finished, the inner temperature was raised up to 25°C to conduct the reaction for 16 hours. The temperature was then raised to 40°C and the reaction was further conducted for another 2 hours. After the reaction has been finished, 39 ml of the tetrahydrofurah was distilled off under'a reduced pressure.

To the obtained slurry solution was added 100 ml of isopropyl alcohol, and the distillation was further effected under a reduced pressure until the solvents no longer flew. The amounts of the solvents distilled off were 49 ml of tetrahydrofuran and 95 ml of isopropyl alcohol. To the obtained residue was again added 100 ml of isopropyl alcohol, and the mixture was stirred at 0°C for one hour. The crystals were separated by using a small centrifugal filtering machine, and were washed twice with 100 ml of isopropyl alcohol and were dried under a reduced pressure to obtain 72.2 g of the 2-(2-chloroacetylaminothiazole-4-yl)-2-(Z)-methoxyiminoacetic acid. The yield was 86.6%.

The product was analyzed by HPLC and it was found that the purity was 99.46% containing impurities, i.e., 2-(2-chloroacetylaminothiazole-4-yl)-2-(E)-methoxyiminoacetic acid in an amount of 0.02% and chloroacetyldithiazole derivative in an amount of 0.04%.

### (Comparative Example 1)

60 Milliliters of a triethylamine was introduced into a 200-ml eggplant-type flask equipped with a dryer tube filled with calcium chloride, and was cooled down to -5°C, to which was then added 20.1 g (0.1 mol) of the same 2-(2-aminothiazole-4-yl)-2-(Z)-methoxyiminoacetic acid as the one used in Example 1, and to which was further dropwisely added 13.6 g (0.12 mols) of the chloroacetyl chloride maintaining the temperature of the reaction solution at not higher than 0°C. Thereafter, the mixture was stirred for one hour. The reaction solution became coal-black and could no longer be stirred. The tar-like substance was separated and analyzed by HPLC. The conversion of the reaction was as small as 9.4%, and the 2-(2-chloroacetylaminothiazole-4-yl)-2-(Z)-methoxyiminoacetic acid had been formed in an amount of as small as 7.80%.

### (Comparative Example 2)

42.0 Grams (0.21 mols) of the same 2-(2-aminothiazole-4-yl)-2-(Z)-methoxyiminoacetic acid as the one used in Example 1, 60 ml of the tetrahydrofuran and 39.9 g (0.50 mols) of a pyridine were added into a 500-ml four neck distillation flask equipped with a stirrer and a dryer tube filled with calcium chloride, and were cooled down to -5°C. After cooling, 28.5 g (0.25 mols) of the chloroacetyl chloride was slowly and dropwisely added over a period of one hour so that the inner temperature was not higher than 0°C. After the dropwise addition has been finished, the reaction was conducted at 0°C for 18 hours. Analysis by the HPLC indicated that the conversion of the reaction was 49.6% and formation of the 2-(2-chloroacetylaminothiazole-4-yl)-2-(Z)-methoxyiminoacetic acid which is the object product was 42.1%. At the same time, however, a 2-(2-chloroacetylaminothiazole-4-yl)-2-(E)-methoxyiminoacetic acid which is an isomer had been by-produced in an amount of 3.1%.

The temperature of the reaction solution was returned to room temperature and THF was distilled off under a reduced pressure. The mixture solution was then dispersed in 600 ml of ethyl acetate and was transferred into a 2-liter beaker. Thereafter, 200 ml of the ion-exchanged water was added, and the water-soluble components were removed by separating the solution. The precipitated crystals were separated by filtration. The obtained solution was washed twice with 200 ml of an aqueous solution having a pH of 1 that had been cooled to 5°C. Then, ethyl acetate was distilled off under a reduced pressure, and 200 ml of methylene chloride was added to separate the precipitated crystals by filtration.

The obtained crystals were dried under a reduced pressure to obtain 23.3 g of the 2-(2-chloroacetylaminothiazole-4-yl)-2-(Z)-methoxyiminoacetic acid. The yield was 40.0%.

The obtained product was analyzed by HPLC and it was found that the purity was 90.1% containing impurities, i.e., 2-(2-chloroacetylaminothiazole-4-yl)-2-(E)-methoxyiminoacetic acid in an amount of 6.9% and chloroacetyldithiazoleacetic acid derivative in an amount of 0.4%. The starting material had remained in an amount of 2.0%.

### (Comparative Example 3)

60 Milliliters of an N,N-dimethylacetamide was introduced into a 100-ml eggplant-type flask and was cooled down to -15°C, followed by the addition of 45.9 g (0.2 mols) of an ester of ethyl 2-(2-aminothiazole-4-yl)-2-(Z)-methoxyiminoacetate. After the liquid temperature was cooled down to not higher than -5°C, 27.1 g (0.24 mols) of the chloroacetyl chloride was dropwisely added thereto such that the temperature of the reaction solution did not exceed 0°C. The reaction was conducted for 3 hours while maintaining the temperature of the solution not to exceed 0°C. Thereafter, the reaction solution was added to 200 ml of the ion-exchanged water and was extracted twice with 200 ml of ethyl acetate.

The extract was washed four times with 100 ml of the ion-exchanged water, ethyl acetate was distilled off under a reduced pressure, and 100 ml of hexane was added to effect the filtration. The obtained crystals were dried under a reduced pressure to obtain 48.0 g of an ester of an ethyl 2-(2-chloroacetylaminothiazole-4-yl)-2-(Z)-methoxyiminoacetate. The yield was 78.5%. The obtained product was analyzed by HPLC and it was found that the purity was 99.87%.

100 Milliliters of ethanol was added to a solution obtained by suspending 30.6 g (0.1 mol) of the above ester of ethyl 2-(2-chloroacetylaminothiazole-4-yl)-2-(Z)-methoxyiminoacetate in 125 ml of a 1N sodium hydroxide aqueous solution, and the mixture was stirred at room temperature for 15 hours. By using 10% hydrochloric acid, the pH of the reaction was set to be 7, and ethanol was distilled off under a reduced pressure. After the water layer was washed with ethyl acetate, the pH was set to be 2 with 10% hydrochloric acid, and the solution was stirred while being cooled with ice. An oily semi-solid product has precipitated.

The semi-solid product was separated by filtering, washed with acetone, and was recrystallized from ethanol to obtain 8.2 g (yield 29.6%) of the 2-(2-chloroacetylaminothiazole-4-yl)-2-(Z)-methoxyiminoacetic acid in a red solid form.

The obtained product was analyzed by HPLC and it was found that the purity was 81.21% containing impurities, i.e., 2-(2-ethoxyacetylaminothiazole-4-yl)-2-(Z)-methoxyiminoacetic acid in an amount of 17.10%. 1.00 Gram of the 2-(2-aminothiazole-4-yl)-2-(Z)-methoxyiminoacetic acid was dissolved by adding, thereto, 30 ml of ethyl acetate and 0.95 ml of ion-exchanged water. Then, 30 ml of hexane was added thereto, and the mixture was cooled down to -25°C to recover 0.52 g of a product by recrystallization.

The recovered product was analyzed by HPLC and it was found that the purity was 83.01% containing impurities, i.e., 2-(2-ethoxyacetylaminothiazole-4-yl)-2-(Z)-methoxyiminoacetic acid in an amount of as large as 16.11%. Impurities could not be removed by recrystallization.

### (Comparative Example 4)

30.57 Grams (0.1 mol) of the ester of the ethyl 2-(2-chloroacetylaminothiazole-4-yl)-2-(Z)-methoxyiminoacetate synthesized by the method of Comparative Example 3 was dissolved in 1.3 liters of ethanol, followed by the addition of a solution obtained by dissolving 28.00 g (0.5 mols) of potassium hydroxide in 250 ml of the ion-exchanged water. The mixture was stirred at room temperature for 2 hours.

Ethanol was distilled off under a reduced pressure, 250 ml of water was added thereto, followed by washing with 300 ml of ethyl acetate. Then, the water layer was adjusted for its pH to be 2 with 10% of hydrochloric acid, and was extracted twice each with 440 ml of ethyl acetate. The extracts were combined together and were washed with saturated saline solution and dried. Then, the solvent was distilled off to obtain 9.5 g (yield, 34.1%) of the 2-(2-chloroacetylaminothiazole-4-yl)-2-(Z)-methoxyiminoacetic acid in a fine yellow powdery form. The obtained product was analyzed with HPLC and it was found that the purity was 80.03% containing impurities, i.e., 2-(2-ethoxyacetylaminothiazole-4-yl)-2-(Z)-methoxyiminoacetic acid in an amount of 17.81%. 1.00 Gram of the 2-(2-aminothiazole-4-yl)-2-(Z)-methoxyiminoacetic acid was dissolved by adding, thereto, 30 ml of ethyl acetate and 0.95 ml of ion-exchanged water. Then, 30 ml of hexane was added thereto, and the mixture was cooled down to -25°C to recover 0.50 g of a product by recrystallization.

The recovered product was analyzed by HPLC and it was found that the purity was 81.29% containing impurities, i.e., 2-(2-ethoxyacetylaminothiazole-4-yl)-2-(Z)-methoxyiminoacetic acid in an amount of as large as 16.42%. Impurities could not be removed by recrystallization.

## Claims

1. A process for producing a chloroacetylaminothiazoleacetic acid derivative of the following formula (II) 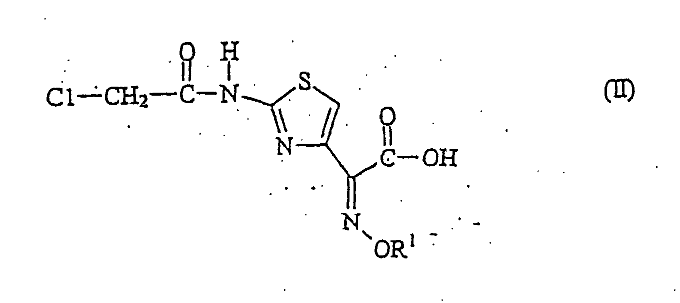 wherein R¹ is a methyl protection group which prevents a hydroxyl group from reacting with an agent for chloroacetylating the amino group, which comprises reacting in the presence of an acid-trapping agent an aminothiazoleacetic acid derivative of the following formula (I) 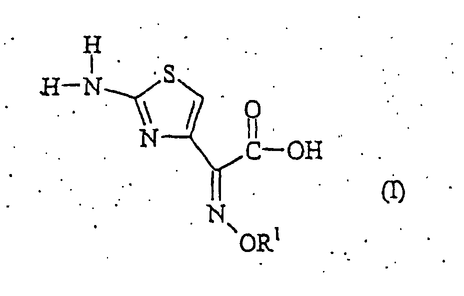 wherein R¹ is as defined above, with the agent for chloroacetylating the amino group;
**characterised in that** the acid-trapping agent is chosen from N,N-dimethylformamide, N,N-dimethylacetamide, N,N-diethylacetamide, N-methylpyrrolidone, N-ethylpyrrolidone, 4-pyrimidone, N-methylmaleimide, N-methylsuccinimide, 12-crown-4 ether, 15-crown-5 ether, 18-crown-6 ether, 1,2,dimethoxyethane, dimethyldiethylene glycol, dimethylpolyethylene glycol, dimethyltripropylene glycol, polyethylene glycol, ethylene oxide, propylene oxide, 1,2-epoxybutane, 1,2-epoxypentane, 1,2-epoxyhexane, 1,2-epoxyheptane, 1,2-epoxyoctane, 1,2-epoxynonane, 1,2-epoxydecane, 1,2-epoxydodecane, 1,2-epoxytetradecane, 1,2-epoxyhexadecane, 1,2-epoxyoctadecane, 1,2-epoxycyclopentane, 1,2-epoxycyclohexane, 1,4-epoxycyclohexane, 3,4-epoxy-1-butene, 3,4-epoxycycloehexylmethyl 3,4-epoxycyclohexanecarboylate, 1,2-epoxy-9-decane, 3-epoxyethyl-7-oxabicyclo[4,1,0] heptane, 1,2-epoxy-5-hexene, 1,2-epoxy-7-octene, exo-2,3-epoxynorbornane, exo-3,6-epoxy-1,2,3,6-tetrahydrophthalic acid anhydride, 1,2-epoxy-3-phenoxypropane, (2,3-epoxypropyl) benzene, 2,3-epoxypropyl furfuryl ether, 2,3-epoxypropyl methacrylate, 2,3-epoxypropyl 4-methoxyphenyl ether, N-(2,3-epoxypropyl)phthalimide, 1,4-epoxy-1,2,3,4-tetrahydronaphthalene, 3,4-epoxytetrahydrothiophene-1,1-dioxide, epichlorohydrin, epibromohydrin, epifluorohydrin, styrene oxide, isobutylene, butadiene, isoprene, styrene, 2,3-dihydofuran, 2,5-dihydrofuran, 4,5-dihydro-2-methylfuran, 3,4-dihydro-2H-pyran, 5,6-dihydro-2H-pyran-2-one, 3,4-dihydro-2-methoxy-2H-pyran or 3,4-dihydro-4-methyl-2H-pyran.

2. A process according to claim 1, which additionally comprises producing the derivative of formula (I) by transforming an ester of an aminothiazoleacetic acid derivative of the following formula (III) 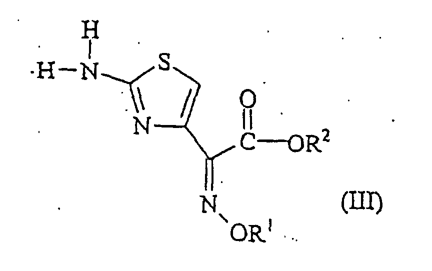 wherein R¹ is as defined above, and R² is an aralkyl or cyclic alkyl or straight or branched chain alkyl protection group for the carboxyl group.

3. A process according to any preceding claim, wherein said acid-trapping agent is used in an amount of not smaller than 1.0 equivalent with respect to the acid by-produced during the process.

4. A process according to any preceding claim in which for R² the alkyl group has 8 or fewer carbon atoms; and the aralkyl group has 4 or fewer rings and an alkyl group of three or fewer carbon atoms.

5. A process according to claim 4, in which the alkyl group is a straight or branched chain alkyl group having 4 or fewer carbon atoms or a cyclic alkyl group having 5 or 6 carbon atoms; and the aralkyl group has 3 or fewer rings and a methyl group.

## Patentansprüche

1. Verfahren zur Herstellung eines Chloracetylaminothiazolessisäure-Derivats der folgenden Formel (II) 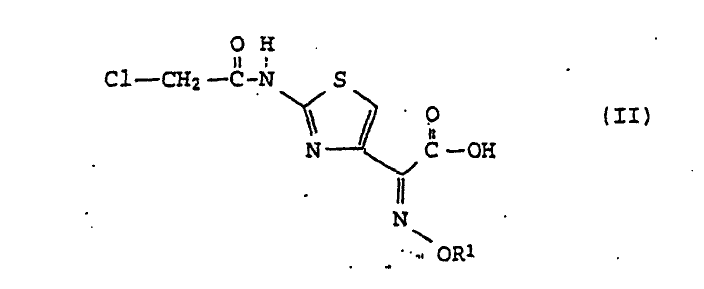 worin R¹ eine Methylschutzgruppe bedeutet, die eine Hydroxylgruppe an einer Umsetzung mit einem Mittel zur Chloracetylierung der Aminogruppe hindert,
wobei das Verfahren die Umsetzung eines Aminothiazolessigsäure-Derivats der folgenden Formel (I) 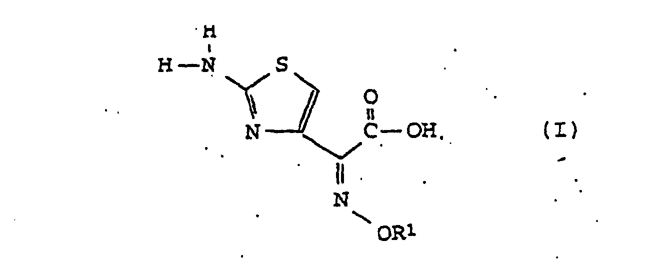 worin, R¹ die vorstehend definierte Bedeutung hat,
in Gegenwart eines Säureabfangmittels mit einem Mittel zur Chloracetylierung der Aminogruppe umfasst, **dadurch gekennzeichnet, dass** das Säureabfangmittel ausgewählt ist unter: N,N-Dimethylformamid, N,N-Dimethylacetamid, N,N-Diethylacetamid, N-Methylpyrrolidon, N-Ethylpyrrolidon, 4-Pyrimidon, N-Methylmaleinimid, N-Methylsuccinimid, 12-Krone-4-ether, 15-Krone-5-ether, 18-Krone-6-ether, 1,2-Dimethoxyethan, Dimethyldiethylenglykol, Dimethylpolyethylenglykol, Dimethyltripropylenglykol, Polyethylenglykol, Ethylenoxid, Propylenoxid, 1,2-Epoxybutan, 1,2-Epoxypentan, 1,2-Epoxyhexan, 1,2-Epoxyheptan, 1,2-Epoxyoctan, 1,2-Epoxynonan, 1,2-Epoxydecan, 1,2-Epoxydodecan, 1,2-Epoxytetradecan, 1,2-Epoxyhexadecan, 1,2-Epoxyoctadecan, 1,2-Epoxycyclopentan, 1,2-Epoxycyclohexan, 1,4-Epoxycyclohexan, 3,4-Epoxy-1-buten, 3,4-Epoxycyclohexylmethyl, 3,4-Epoxycyclohexancarboxylat, 1,2-Epoxy-9-decen, 3-Epoxyethyl-7-oxabicyclo[4,1,0]heptan, 1,2-Epoxy-5-hexen, 1,2-Epoxy-7-octen, Exo-2,3-epoxynorbornan, Exo-3,6-epoxy-1,2,3,6-tetrahydrophthalsäureanhydrid, 1,2-Epoxy-3-phenoxypropan, (2,3-Epoxypropyl)-benzol, 2,3-Epoxypropylfurfurylether, 2,3-Epoxypropylmethacrylat, 2,3-Epoxypropyl-4-methoxyphenylether, N-(2,3-Epoxypropyl)-phthalimid, 1,4-Epoxy-1,2,3,4-tetrahydronaphthalin, 3,4-Epoxytetrahydrothiophen-1,1-dioxid, Epichlorhydrin, Epibromhydrin, Epifluorhydrin, Styroloxid, Isobutylen, Butadien, Isopren, Styrol, 2,3-Dihydrofuran, 2,5-Dihydrofuran, 4,5-Dihydro-2-methylfuran, 3,4-Dihydro-2H-pyran, 5,6-Dihydro-2H-pyran-2-on, 3,4-Dihydro-2-methoxy-2H-pyran oder 3,4-Dihydro-4-methyl-2H-pyran.

2. Verfahren nach Anspruch 1, das zusätzlich die Herstellung des Derivats der Formel (I) durch Umwandeln eines Esters eines Aminothiazolessigsäure-Derivats der folgenden Formel (III) umfasst 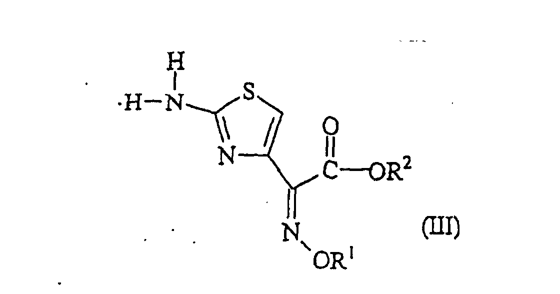 worin R¹ die vorstehend definierte Bedeutung hat und R² eine Aralkyl- oder Cycloalkyl- oder geradkettige Alkyl-Schutzgruppe für die Carboxylgruppe bedeutet.

3. Verfahren nach einem der vorstehenden Ansprüche, wobei das Säureabfangmittel in einer Menge von nicht unter 1,0 Äquivalent, bezogen auf die während des Verfahrens als Nebenprodukt gebildete Säure, verwendet wird.

4. Verfahren nach einem der vorstehenden Ansprüche, wobei im Rahmen von R² die Alkylgruppe 8 oder weniger Kohlenstoffatome und
die Aralkylgruppe 4 oder weniger Ringe und die Alkylgruppe 3 oder weniger Kohlenstoffatome aufweist.

5. Verfahren nach Anspruch 4, wobei es sich bei der Alkylgruppe um eine geradkettige Alkylgruppe mit 4 oder weniger Kohlenstoffatomen oder um eine cyclische Alkylgruppe mit 5 oder 6 Kohlenstoffatomen handelt und
die Aralkylgruppe 3 oder weniger Ringe und eine Methylgruppe aufweist.

## Revendications

1. Procédé pour la préparation d'un dérivé d'acide chloracétylaminothiazolacétique ayant la formule (II) suivante 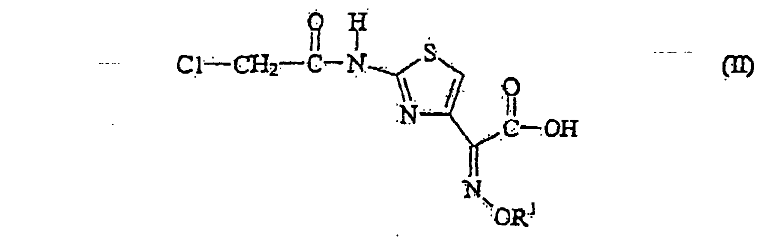 dans laquelle R¹ est un groupe de protection méthyle qui empêche un groupe hydroxyle de réagir avec un agent de chloracétylation du groupe amino,
comprenant la mise en réaction, en présence d'un agent piégeant les acides, d'un dérivé de l'acide aminothiazolacétique ayant la formule (I) suivante 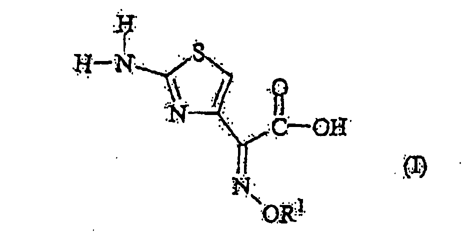 dans laquelle R¹ est tel que défini ci-dessus, avec l'agent de chloracétylation du groupe amino ;
**caractérisé en ce qu'**on choisit l'agent piégeant les acides parmi les N,N-diméthylformamide, N,N-diméthyl-acétamide, N,N-diéthylacétamide, N-méthylpyrrolidone, N-éthylpyrrolidone, 4-pyrimidone, N-méthylmaléimide, N-méthylsuccinimide, éther 12-couronne-4, éther 15-couronne-5, éther 18-couronne-6, 1,2-diméthoxyéthane, diméthyldiéthylèneglycol, diméthylpolyéthylèneglycol, diméthyltripropylèneglycol, polyéthylèneglycol, oxyde d'éthylène, oxyde de propylène, 1,2-époxybutane, 1,2-époxypentane, 1,2-époxyhexane, 1,2-époxyheptane, 1,2-époxyoctane, 1,2-époxynonane, 1,2-époxydécane, 1,2-époxydodécane, 1,2-époxytétradécane, 1,2-époxyhexadécane, 1,2-époxyoctadécane, 1,2-époxycyclopentane, 1,2-époxycyclohexane, 1,4-époxycyclohexane, 3,4-époxy-1-butène, 3,4-époxycyclohexylméthyle, carboxylate de 3,4-époxycyclohexane, 1,2-époxy-9-décène, 3-époxyéthyl-7-oxabicyclo[4,1,0]heptane, 1,2-époxy-5-hexène, 1,2-époxy-7-octène, exo-2,3-époxynorbornane, anhydride de l'acide exo-3,6-époxy-1,2,3,6-tétrahydrophtalique, 1,2-époxy-3-phénoxypropane, (2,3-époxypropyl)benzène, éther de 2,3-époxypropylfurfuryle, méthacrylate de 2,3-époxypropyle, éther 2,3-époxypropyl-4-méthoxyphénylique, N-(2,3-époxypropyl)phtalimide, 1,4-époxy-1,2,3,4-tétrahydronaphtalène, 3,4-époxytétrahydrothiophène-1,1-dioxyde, épichlorhydrine, épibromhydrine, épifluorhydrine, oxyde de styrène, isobutylène, butadiène, isoprène, styrène, 2,3-dihydrofuranne, 2,5-dihydrofuranne, 4,5-dihydro-2-méthylfuranne, 3,4-dihydro-2H-pyranne, 5,6-dihydro-2H-pyranne-2-one, 3,4-dihydro-2-méthoxy-2H-pyranne ou 3,4-dihydro-4-méthyl-2H-pyranne.

2. Procédé selon la revendication 1, comprenant, en outre, la préparation du dérivé de formule (I) en transformant un ester d'un dérivé d'acide aminothiazolacétique ayant la formule (III) suivante 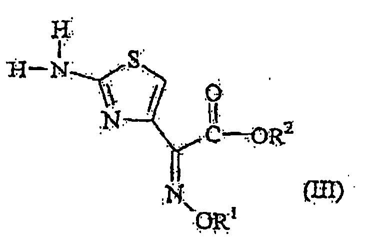 dans laquelle R¹ est tel que défini ci-dessus, et R² est un groupe de protection aralkyle ou alkyle cyclique ou alkyle à chaîne linéaire pour le groupe carboxyle.

3. Procédé selon l'une quelconque des revendications précédentes, dans lequel on utilise ledit agent piégeant les acides en une quantité qui n'est pas inférieure à 1,0 équivalent par rapport à l'acide produit en tant que produit secondaire pendant le procédé.

4. Procédé selon l'une quelconque des revendications précédentes, dans lequel, pour R² :
le groupe alkyle a 8 atomes de carbone ou moins ; et
le groupe aralkyle a 4 noyaux ou moins et un groupe alkyle ayant trois atomes de carbone ou moins.

5. Procédé selon la revendication 4, dans lequel le groupe alkyle est un groupe alkyle à chaîne linéaire ayant 4 atomes de carbone ou moins ou un groupe alkyle cyclique ayant 5 ou 6 atomes de carbone ; et
le groupe aralkyle a 3 noyaux ou moins et un groupe méthyle.
